# EUROPÄISCHE PATENTANMELDUNG

(11) **EP 1 125 944 A1**
(43) Veröffentlichungstag der Anmeldung: **22.08.2001**
(21) Anmeldenummer: 00103540.1
(22) Anmeldetag: 18.02.2000
(51) Int. Cl.: C07H 17/04, C12P 19/60, C12P 19/26, A61K 31/70, A61P 35/00

(54) **Pluraflavine und Derivate davon, Verfahren zu ihrer Herstellung und Verwendung derselben**

(71) Anmelder: Aventis Pharma Deutschland GmbH, 65929 Frankfurt am Main (DE)
(72) Erfinder: Vertésy, Laszlo, Dr., 65817 Eppstein-Vockenhausen (DE); Ehrlich, Klaus, Dr., 65428 Rüsselsheim (DE); Knauf, Martin, Dr., 60529 Frankfurt (DE); Wink, Joachim, Dr., 63322 Rödemark (DE); Barbone, Francis P., Annandale, NJ 08801 (US); Powers, Elaine A., High Bridge NJ 08829 (US); Cashman, Elizabeth A., Bridgewater NJ 08807 (US)

(57) **Zusammenfassung**

Die Erfindung betrifft Verbindungen der allgemeinen Formel I 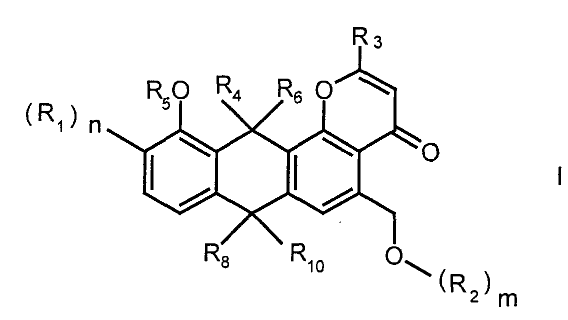 worin
R₁ und R₂ unabhängig von einander jeweils einen Zucker bedeuten; R₃ eine Epoxid-enthaltende Gruppe, oder C₁-C₆-Alkyl oder C₂-C₆-Alkenyl, gegebenenfalls mit einem oder mehreren OH substituiert, bedeutet; R₅ H, C₁-C₆-Alkyl, C₂-C₆-Alkenyl oder C₂-C₆-Alkinyl bedeutet; R₄, R₆, R₈ und R₁₀, können, unabhängig von einander, H, C₁-C₆-Alkyl, C₂-C₆-Alkenyl, C₂-C₆-Alkinyl, -X₂H, oder -X₂R₁₂ bedeuten, oder R₄ und R₆ können zusammen und/oder R₈ und R₁₀ zusammen =X₂ sein;
X₂ kann O, NH, N-C₁-C₆ Alkyl, N-C₂-C₆-Alkenyl, N-C₂-C₆-Alkinyl oder S bedeuten;
R₁₂ C₁-C₆-Alkyl, C₂-C₆-Alkenyl, C₂-C₆-Alkinyl, Aryl oder Acyl bedeutet; und
m und n 1 oder 2; in allen stereochemischen Formen und Gemische dieser Formen in allen Verhältnissen, sowie deren physiologisch verträglichen Salze. Die Verbindungen sind erhältlich aus Kulturen des Mikroorganismus *Actinomycetales* species HAG 003959, DSM 12931, durch Fermentation. Die Erfindung betrifft somit ein Verfahren zur deren Herstellung und die Verwendung der Verbindungen als Arzneimittel, insbesondere als Antitumor Mittel.

## Beschreibung

Die vorliegende Erfindung betrifft neue Verbindungen der allgemeinen Formel I 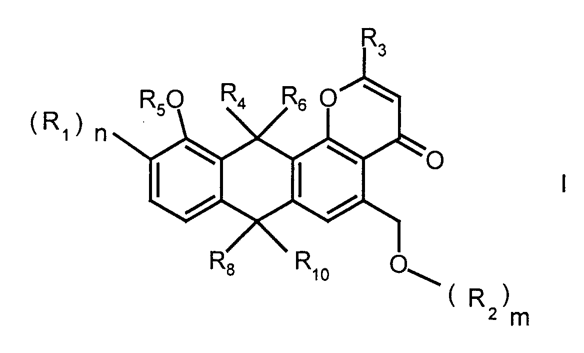 worin R₁, R₂, R₃, R₄, R₅, R₆, R₈, R₁₀, m und n die Bedeutung wie unten beschrieben haben. Die Verbindungen der Formel I hemmen die Transkriptase, besitzen cytostatische Wirkungen und sind besonders geeignet zur Behandlung von Tumoren. Die Verbindungen der Formel I sind erhältlich durch Fermentierung von *Actinomycetales* species HAG 003959, DSM 12931 in einem Kulturmedium. Die Erfindung betrifft somit ein Verfahren zur Herstellung der Verbindungen der Formel I, die Verwendung der Verbindungen zur Herstellung eines Arzneimittels zur Behandlung von malignen Erkrankungen und von Krankheiten die durch hemmen von Transkriptase Behandelt werden können, sowie pharmazeutischen Zubereitungen mit einem Gehalt an mindestens einer Verbindung der Formel I.

Krebs ist eine zumeist tödlich verlaufende Erkrankung von Mensch und Tier, die durch das unkontrollierte Wachsen von körpereigenen Zellen verursacht wird. Krebs ist die Bezeichnung für die Bildung von bösartigen Geschwülsten (Malignome), von Neoplasmen (Tumoren bzw. Carcinoma) oder für die maligne Entartung sowie Reifungsstörung weißer Blutzellen (Leukämie, Blutkrebs). Krebs- oder Tumorzellen entstehen durch Umwandlung körpereigener Zellen. Die Bösartigkeit der Krebszelle drückt sich in der Autonomie des Wachstums aus, das heißt in ihrer Fähigkeit, ungehemmt und ohne Einordnung in den Bauplan der Organe und unter Gewebszerstörung infiltrierend zu wachsen. Ein sicheres Zeichen der Malignität ist die Bildung tumorferner Absiedlungen (Metastasen) nach hämatogener oder lymphogener Ausbreitung von Tumorzellen. Krebs gehört zu den häufigsten Todesursachen des Menschen und deshalb besteht ein großer Bedarf an Methoden und Mitteln zur Heilung oder Behandlung von malignen Entartungen.

Die Möglichkeit einer Therapie maligner Tumoren umfaßt neben der - wenn möglich radikalen - operativen Entfernung des Tumors, die radiologische Therapie mit Röntgenstrahlen, α-, β-, γ-Strahlen, die Immuntherapie und die Chemotherapie. Die Immuntherapie ist derzeit nur in eingeschränktem Maß anwendbar. Unter der Chemotherapie von Tumoren versteht man die Gabe von Zellgiften (Cytostatika) zur Behandlung von Tumoren und von verbliebenen Tumorzellen nach lokaler chirurgischer Behandlung oder Bestrahlung. Diese Stoffe greifen spezifisch in bestimmte Vorgänge der Zellteilung ein, so daß Gewebe mit hohem Anteil an sich teilenden Zellen, wie das rasch wachsende Tumorgewebe, empfindlicher reagieren. Zum Einsatz kommen alkylierende Verbindungen, wie z. B. das Cyclophosphamid (The Merck Index, 12. Ed. Seite 463), Antimetabolite, wie das Methotrexat (The Merck Index, 12. Ed. Seite 1025), Alkaloide, wie das Vincristin (The Merck Index, 12. Ed. Seite 1704) und Antibiotika, wie das Daunomycin (The Merck Index, 12. Ed. Seite 479) sowie das Adriamycin (The Merck Index, 12. Ed. Seite 581-582). All diese Agenzien haben aber aufgrund von massiven Nebenwirkungen große Nachteile, so daß der Tod der Erkrankten nur hinausgezögert, nicht jedoch abgewendet wird. Zudem treten bei entarteten (Krebs-) Zellen Resistenzen gegen die angewandten Mittel auf, die derzeitigen Medikamente wirken dann nicht mehr cytostatisch, wohl aber toxisch infolge der Nebenwirkungen. Zudem hat sich gezeigt, daß eine kombinierte bzw. sequentielle Anwendung von Cytostatika die Wirksamkeit eines einzelnen Cytostatikums (Monotherapie) übertrifft und dadurch möglich ist, daß sich die erheblichen Nebeneffekte bei der Polychemotherapie nicht addieren. Aus all diesen Gründen werden neue Chemotherapeutika dringend benötigt und deshalb weltweit gesucht.

Es ist überraschend gefunden worden, daß der Mikroorganismus-Stamm *Actinomycetales* species HAG 003959, DSM 12931, hoch wirksame neue Cytostatika zu bilden vermag, die das Wachstum von Zellen noch in sehr geringen Konzentrationen hemmen. Die neuen Verbindungen werden in Folgendem Pluraflavine genannt und sind, zusammen mit Pluraflavin- Derivaten, Gegenstand der Erfindung. Die Pluraflavine sind Antibiotika, die aus einem p-chinoiden Ringsystem und verschiedenen Zuckerbausteinen bestehen und die Transkription durch Interkalierung des Nukleinsäure-Doppelstrang sowie gegebenenfalls zusätzliche Alkylierung hemmen. Das Ringgerüst ist erstmals als ein Teil des Pluramycins von S. Kondo et al. im Journal of Antibiotics, Band 30, Seite 1143-1145, 1977 beschrieben worden. Dieses Ringgerüst wurde später in mehreren Antibiotika gefunden: neben dem Pluramycin und Neopluramycin sind die Verbindungen Saptomycine (N. Abe et al. J. Antibiotics, 46, 1536-1549, 1993), Ankinomycin (Y. Sato et al. J. Antibiotics 42, 149, 1989), Kidamycin (N. Kanda et al. J. Antibiotics, 24, 599, 1971), Hedamycin ( U. Sequin et al. Tetrahedron, 34, 761, 1978) sowie die Altromycine (G. M. Brill et al. J. Antibiotics, 43, 229-237, 1990) als strukturverwandte Verbindungen charakterisiert. Die bisher bekannten Substanzen weisen oft Nachteile auf, die sich in unbefriedigender Wirkhöhe, hoher Toxizität und/oder unerwünschten Nebenwirkungen äußern.

Die vorliegende Erfindung betrifft daher Verbindungen der allgemeinen Formel I 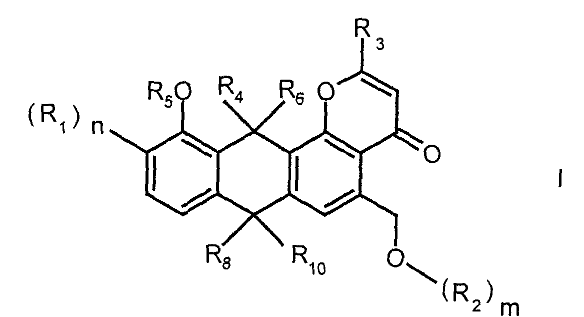 worin bedeuten
R₁ und R₂, unabhängig von einander, jeweils einen Zucker,
R₃ eine Epoxid-enthaltende Gruppe oder
C₁-C₆-alkyl oder C₂-C₆-alkenyl, gegebenenfalls mit einem oder mehreren OH substituiert,
R₅ H, C₁-C₆-Alkyl, C₂-C₆-Alkenyl oder C₂-C₆-Alkinyl,
R₄, R₆, R₈ und R₁₀, unabhängig von einander, H, C₁-C₆-Alkyl, C₂-C₆-Alkenyl, C₂-C₆-Alkinyl, -X₂H, oder -X₂R₁₂ oder
R₄ und R₆ zusammen und/oder R₈ und R₁₀ zusammen =X₂,
X₂ O, NH, N-C₁-C₆-Alkyl, N-C₂-C₆-Alkenyl, N-C₂-C₆-Alkinyl oder S,
R₁₂ C₁-C₆-Alkyl, C₂-C₆-Alkenyl, C₂-C₆-Alkinyl, Aryl oder Acyl, und
m und n, unabhängig von einander, 1 oder 2,
in allen ihren stereochemischen Formen und Gemische dieser Formen in jedem Verhältnis,
sowie deren physiologisch verträgliche Salze.

In der Formel I bedeutet
C₁-C₆-Alkyl ein geradkettiges oder verzweigtes Alkyl mit 1 bis 6 C-Atomen, wie z.B. Methyl, Ethyl, i-Propyl, tert.Butyl und Hexyl,
C₂-C₆-Alkenyl ein geradkettiges oder verzweigtes Alkenyl mit 2 bis 6 C-Atomen, wie z.B. Allyl, Crotyl und Pentenyl, und
C₂-C₆- Alkinyl ein geradkettiges oder verzweigtes Alkinyl mit 2 bis 6 C-Atomen, wie z.B. Propinyl, Butinyl und Pentinyl.

Aryl kann z.B. stehen für Phenyl, Benzyl oder 1- oder 2- Naphthyl, das auch noch substituiert sein kann, beispielsweise durch Halogen, wie Chlor, Brom, Fluor, durch Alkyl mit 1-4 C-Atomen, vorzugsweise Methyl-, durch Hydroxy, durch Alkoxy mit 1-4C Atomen, insbesondere Methoxyl und/oder durch Trifluormethyl.

Acyl kann stehen für aliphatische oder aromatische Acyl-Reste. Das aliphatische Acyl hat 1-7, vorzugsweise 1-4 C Atome, wie z.B. Formyl, Acetyl, Propionyl, Butyryl, Hexanoyl, Acryloyl, Crotonoyl, Propioloyl, das noch weiter substituiert sein kann, beispielsweise durch Halogen, wie Chlor, Brom, Fluor, durch Amino, und/oder durch Alkylamino mit 1-4 C Atomen, vorzugsweise Methyl- oder Ethylamino-Gruppen. Aromatisches Acyl kann z.B. Benzoyl oder Naphthoyl sein, das auch noch weiter substituiert sein kann, beispielsweise durch Halogen, wie Chlor, Brom, Fluor, durch Alkyl mit 1-4 C-Atomen, vorzugsweise Methyl, durch Hydroxy, durch Amino-Gruppen, wie z.B. Ethylamino, oder durch Alkoxy-Gruppen mit 1-7, vorzugsweise 1-4C Atomen, insbesondere Methoxy.

Der Zucker R₁ und/oder R₂ ist vorzugsweise einen Aldohexose.

Vorzugsweise bedeutet m 1.

R₂ kann ein Aminozuckerrest der allgemeinen Formel 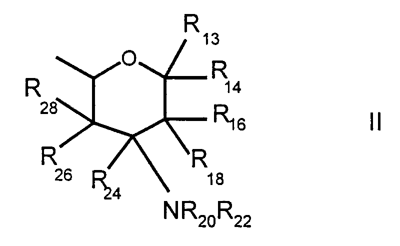 sein, worin R₁₃, R₁₄, R₁₆, R₁₈, R₂₀, R₂₂, R₂₄, R₂₆ und R₂₈, unabhängig von einander, H, OH, NH₂, NHAlkyl, N(Alkyl)₂, Alkoxy sein kann, wobei Alkyl einen C₁-C₄-Rest bedeutet. Beispiele für
Alkyl sind z.B. Methyl, Ethyl, Propyl, Isobutyl und Butyl, insbesondere Methyl, für Alkoxy sind Methoxy, Ethoxy oder Butoxy, insbesondere Methoxy.

Vorzugsweise ist R₂ einer Aminozucker der Formel IIA: 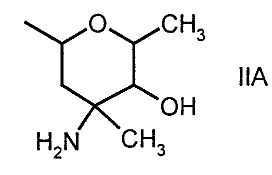 R₁ kann für einen Aminozucker stehen. Vorzugsweise bedeutet n 2.

R₁ kann die allgemeinen Formel III 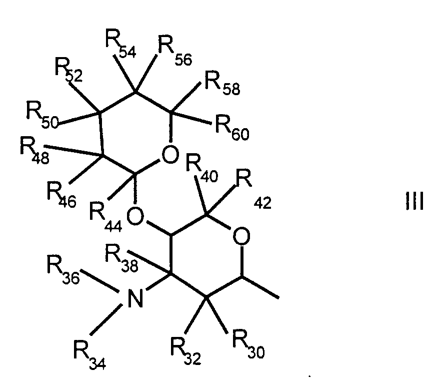 haben, worin R₃₀ bis R₆₀, unabhängig von einander, H, OH, NH₂, NHAlkyl, N(Alkyl)₂, oder Alkoxy sein kann, mit der Maßgabe, daß Alkyl einen C₁-C₄-Rest, wie z.B. Methyl, Ethyl, Propyl, Isobutyl und Butyl, insbesondere Methyl und O-Alkyl, wie z.B. Methoxy, Ethoxy oder Butoxy, insbesondere Methoxy bedeutet.

Vorzuqsweise hat R₁ die Formel IIIA 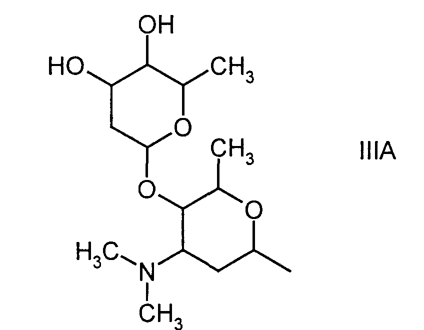 R₃ bedeutet vorzugsweise eine Epoxid-enthaltende-Gruppe. Die Epoxid-enthaltende Gruppe kann ein geradkettiger oder verzweigter Alkyl- oder Alkenyl-Rest mit 2 bis 12, vorzugsweise 2 bis 6 C-Atomen sein, der eine oder zwei Epoxid-Ringe (Oxirane) enthält. Als Beispiele kommen hierfür in Betracht 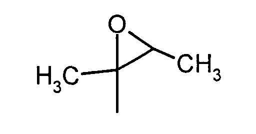 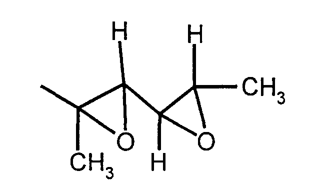 und 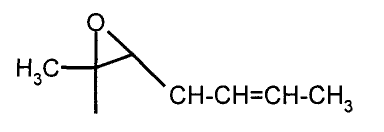 R₃ kann statt einer Epoxid-enthaltenden Gruppe ein geradkettiger oder verzweigter Alkyl mit 1 oder 12, vorzugsweise 1 bis 6 C-Atomen, wie z.B. Methyl, Ethyl, i-Propyl, tert-Butyl, Hexyl, sowie beispielsweise Octyl, Dodecyl, oder ein geradekettiges oder verzweigtes Alkenyl mit 2 bis 12, vorzugsweise 2 bis 6 C-Atomen, wie z.B. Allyl, Crotyl, Pentenyl, sowie Dodecenyl, sein, wobei diese Alkyl- oder Alkenyl- Gruppen noch ein oder mehrfach, substituiert sein können z. B. durch Hydroxy.

Die Erfindung betrifft somit das Pluraflavin A der Formel IA 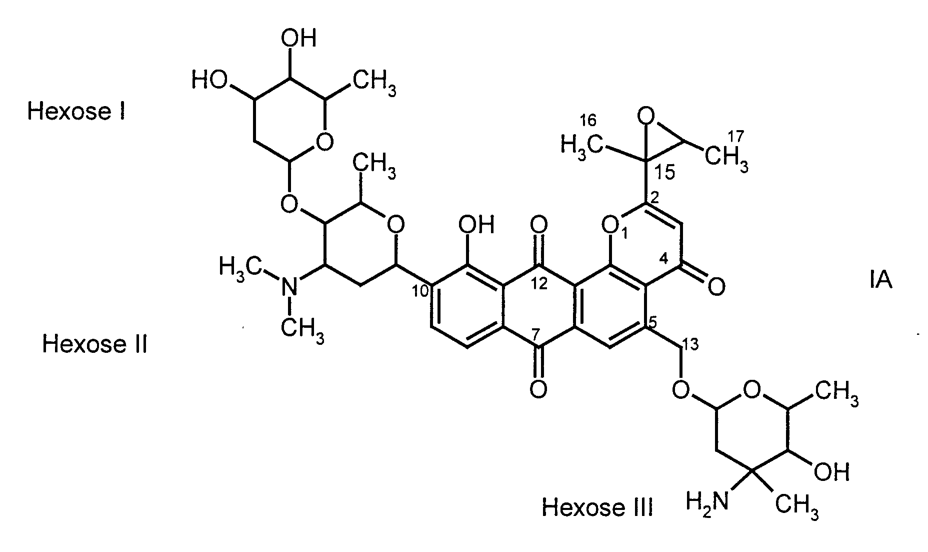 und das Pluraflavin B der Formel IB 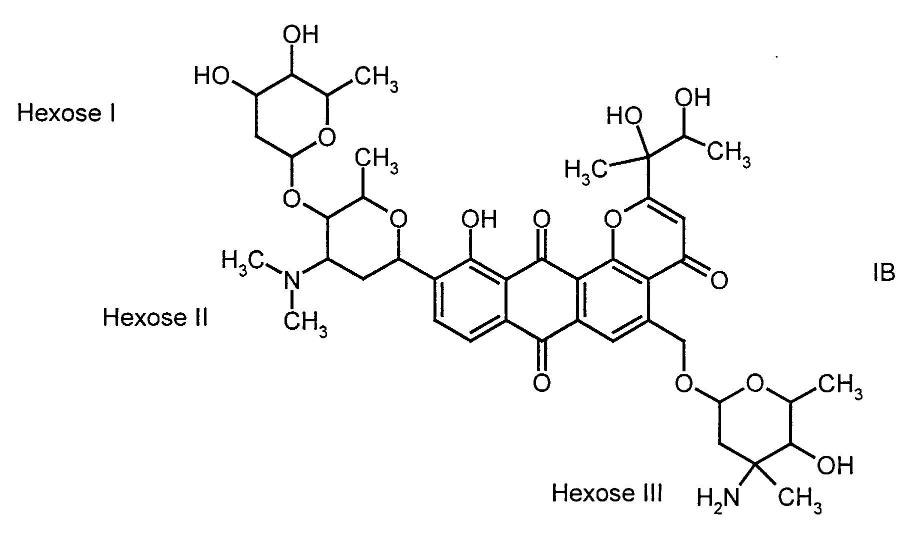
in der Hexose 1 eine 2,6-Didesoxy-aldohexose;
Hexose II eine 2,3,6-Tridesoxy-3-dimethylamino-aldohexose und
Hexose III eine 2,3,6-Tridesoxy-3-amino-3-methyl-aldohexose bedeutet, in allen ihren stereochemischen Formen und Gemische dieser Formen in jedem Verhältnis,
sowie deren physiologisch verträgliche Salze.

Bevorzugt sind die Verbindungen der Formel IA und IB, in denen
Hexose 1 Oliose,
Hexose II Rhodosamin und
Hexose III 3-epi-Vancosamin bedeutet.

Erfindungsgemäß sind die Verbindungen der Formel 1 erhältlich durch Fermentation des *Actinomycetales* species HAG 003959, DSM 12931, oder deren Varianten oder Mutanten unter geeigneten Bedingungen in einem Kulturmedium, bis sich eines oder mehrere der Pluraflavine der Formel IA und/oder IB im Kulturmedium anhäufen. Durch anschließende Isolierung der Verbindungen und gegebenenfalls Umwandlung in chemische Derivate, sowie deren physiologisch verträglichen Salze werden die Pluraflavine gewonnen.

Die Erfindung betrifft daher weiterhin ein Verfahren zur Herstellung einer Verbindung der Formel 1, dadurch gekennzeichnet, daß der Mikroorganismus *Actinomycetales* species HAG 003959, DSM 12931, oder deren Varianten oder Mutanten unter geeigneten Bedingungen in einem Kulturmedium fermentiert wird, bis sich eines oder mehrere der Pluraflavine der Formel IA und/oder IB in dem Kulturmedium anhäufen und anschließend aus dem Kulturmedium isoliert und gegebenenfalls in chemische Derivate und /oder physiologisch verträglichen Salze umgewandelt werden.

Vorzugsweise wird der Stamm HAG 003959, DSM 12931, seine Mutanten und/oder Varianten in einer Nährlösung (auch als Kulturmedium bezeichnet) mit Kohlenstoff- und Stickstoffquelle sowie den üblichen anorganischen Salzen fermentiert, bis sich die neuen Pluraflavine in dem Kulturmedium anhäufen, anschließend werden die Pluraflavine aus dem Kulturmedium isoliert und gegebenenfalls in die einzelnen aktiven Komponenten aufgetrennt.

Die Fermentation wird vorzugsweise unter aeroben Bedingungen durchgeführt, sie verläuft besonders gut bei einer Temperatur zwischen 18 und 35□C und bei einem pH zwischen 6 und 8.

In der Literatur sind viele Reaktionen zur chemischen Abwandlung von Chinonen beschrieben worden. Die Derivatisierung der Chinon-Formen der vorliegenden Verbindungen kann daher mit an sich bekannten chemischen Reaktionen durchgeführt werden. Eine Reduktion zu den Hydrochinon-Formen der Verbindungen kann zum Beispiel mit Wasserstoff katalytisch erreicht werden oder mit Metall-Hydriden, wie Aluminium-Hydriden oder Bor-Hydriden. Als ein weiteres Beispiel kommt die Umsetzung von Chinonen mit Hydroxylamin oder mit seinen Derivaten zu den Oximen in Betracht, die ihrerseits weiter chemisch umgewandelt werden können.

Die Pluraflavin Derivate der Formel IA' und IB' sind von den Pluraflavinen der Formeln IA bzw. IB abgeleitet worden und bilden ebenfalls den Gegenstand dieser Erfindung. 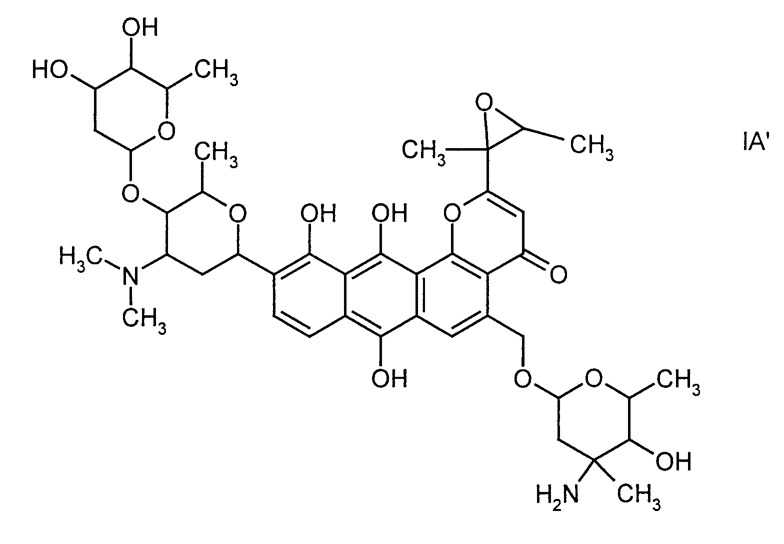 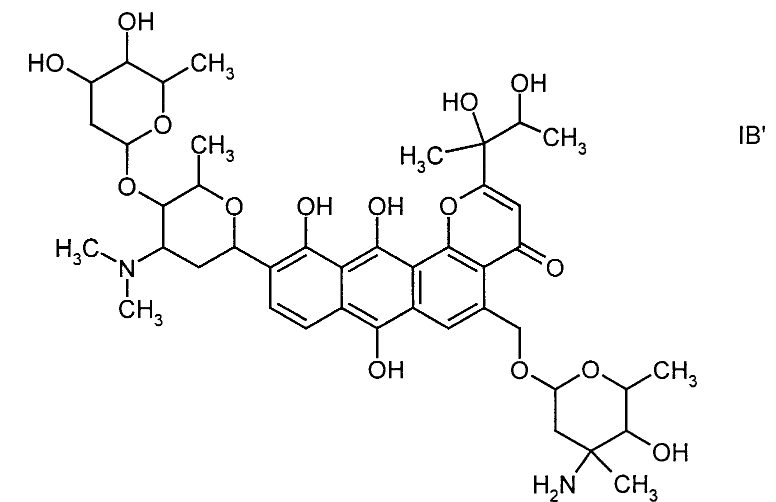 Im folgenden wird die Erfindung detailliert beschrieben, insbesondere in ihren bevorzugten Ausführungsformen.

Die erfindungsgemäßen Pluraflavine werden durch *Actinomycetales* species bevorzugt durch *Actinomycetales* spec HAG 003959, DSM 12931 produziert. *Actinomycetales* species HAG 003959, DSM 12931 besitzt ein beigerotes Mycel und ist durch die für Actinomyceten charakteristischen Conidiophoren gekennzeichnet.

Ein Isolat wurde bei der Deutschen Sammlung von Mikroorganismen und Zellkulturen GmbH, Mascheroder Weg 1B, D 38124 Braunschweig, Deutschland, nach den Regeln des Budapester Vertrages am 16. Juli 1999 unter der folgenden Nummer hinterlegt: *Actinomycetales* species HAG 003959, DSM 12931.

Anstelle des Stammes *Actinomycetales* species HAG 003959, DSM 12931 können auch dessen Mutanten und Varianten eingesetzt werden, die eine oder mehrere Verbindungen der erfindungsgemäßen Pluraflavine synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Das erfindungsgemäße Verfahren kann für die Fermentation im Labormaßstab (Milliliter- bis Literbereich) und für den industriellen Maßstab (Kubikmetermaßstab) eingesetzt werden. Alle Prozentangaben beziehen sich, wenn nichts anderes angegeben ist, auf das Gewicht. Mischungsverhältnisse bei Flüssigkeiten beziehen sich auf das Volumen, wenn keine anderen Angaben gemacht werden.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Pluraflavine verläuft besonders gut in einem Kulturmedium, das etwa 0,1 bis 5 %, bevorzugt 0,3 bis 2 % Glucose und 0,2 bis 5 %, bevorzugt 0,5 bis 3 % Sojamehl und 0,05 bis 2 %, bevorzugt 0,2 bis 1,0 g/l Cornsteep und 0,05 bis 1,0 g/l, bevorzugt 0,1 bis 0,5 % Calciumcarbonat und 0,05 bis 1,0 g/l, bevorzugt 0,1 bis 1,0 g/l Natriumchlorid enthalten. Die Angaben in Prozent sind jeweils bezogen auf das Gewicht des gesamten Kulturmediums.

In dem Kulturmedium bildet *Actinomycetales* species HAG 003959, DSM 12931, ein Gemisch aus Pluraflavinen. Je nach Zusammensetzung des Kulturmediums kann der mengenmäßige Anteil eines oder mehrerer der erfindungsgemäßen Pluraflavine variieren. Außerdem kann durch die Medienzusammensetzung die Synthese einzelner Pluraflavine gesteuert werden, so daß eines oder auch mehrere der Pluraflavine gar nicht bzw. in einer Menge unterhalb der Nachweisgrenze vom Mikroorganismus hergestellt werden.

Vorzugsweise enthält die Kultur ein nachweisbares Pluraflavin. Bevorzugt werden die Pluraflavine A oder B gebildet.

Neben den Pluraflavinen A und B (Verbindungen der Formel IA bzw. IB) werden in dem Kulturmedium des *Actinomycetales* species HAG 003959, DSM 12931, auch weiter verwandte Verbindungen gebildet, die sich durch eine veränderte Glycosylierung von den in den Formeln IA und IB wiedergegebenen Verbindungen unterscheiden. So wurde als Nebenprodukt ein weiteres Pluraflavin mit dem Molekulargewicht 974 Da sowie ein Abbauprodukt des Pluraflavin A, Molekulargewicht 692,77, C₃₇H₄₄N₂O₁₁, nachgewiesen. In Letzterem fehlt die Hexose 1, durch saure Bedingungen kann aus Pluraflavin A die 2,6-Didesoxy-aldohexose hydrolytisch abgespalten werden.

Die Kultivierung des Mikroorganismus erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35°C, vorzugsweise bei etwa 25 bis 32°C, insbesondere bei 27 bis 30°C durchgeführt werden. Der pH-Bereich sollte zwischen 6 und 8 liegen, vorzugsweise zwischen 6,5 und 7,5. Man kultiviert den Mikroorganismus unter diesen Bedingungen im allgemeinen über einen Zeitraum von 24 bis 300 Stunden, vorzugsweise 36 bis 140 Stunden.

Vorteilhaft kultiviert man in mehreren Stufen, d. h. man stellt zunächst eine oder mehrere Vorkulturen in einem flüssigen Kulturmedium her, die dann in das eigentliche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 96 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Malz-Hefe-Agar oder Haferflocken-Agar wachsen läßt.

Der Fermentationsverlauf kann anhand des pH-Wertes der Kulturen oder des Mycelvolumens sowie durch chromatographische Methoden, wie z.B. Dünnschichtchromatographie oder High Pressure Liquid Chromatography oder Ausprüfen der biologischen Aktivität überwacht werden. Die erfindungsgemäßen Pluraflavine sind sowohl im Mycel als auch im Kulturfiltrat enthalten. Das im folgenden beschriebene Isolierungsverfahren dient zur Aufreinigung der erfindungsgemäßen Pluraflavine, vorzugsweise zur Aufreinigung der Pluraflavine A und B.

Die Isolierung bzw. Aufreinigung der erfindungsgemäßen Pluraflavine aus dem Kulturmedium erfolgt nach bekannten Methoden unter Berücksichtigung der chemischen, physikalischen und biologischen Eigenschaften der Naturstoffe. Zum Testen der Pluraflavine-Konzentration im Kulturmedium oder in den einzelnen Isolierungsstufen kann die Dünnschichtchromatographie, beispielsweise an Kieselgel mit Chloroform-Methanol-Eisessig-Wasser - Gemischen (z. B. im Mengenverhältnis 8:1:1:0,2) als Laufmittel oder HPLC verwendet werden. Die Detektion bei der dünnschichtchromatographischen Auftrennung kann beispielsweise durch Färbereagentien wie α-Naphthol/Schwefelsäure erfolgen, wobei die Menge der gebildeten Substanz zweckmäßig mit einer Eichlösung verglichen wird.

Zur Isolierung der erfindungsgemäßen Pluraflavine wird das Mycel zunächst von dem Kulturmedium nach den üblichen Verfahren abgetrennt und anschließend werden die Pluraflavine von der Zellmasse mit einem gegebenenfalls mit Wasser mischbaren organischen Lösungsmittel extrahiert. Die organische Lösungsmittelphase enthält die erfindungsgemäßen Pluraflavine, sie werden gegebenenfalls im Vakuum konzentriert und wie unten beschrieben weiter aufgereinigt.

Das Kulturfiltrat wird gegebenenfalls mit dem Konzentrat des Mycel-Extraktes vereinigt und mit einem geeigneten, mit Wasser nicht mischbarem organischen Lösungsmittel, zum Beispiel mit n-Butanol, extrahiert. Die anschließend abgetrennte organische Phase wird ggf. im Vakuum konzentriert. Man kann das Konzentrat zum entfetten des Wertproduktes mit einem unpolaren Lösungsmittel, in dem die erfindungsgemäßen Pluraflavine sehr wenig löslich sind, wie zum Beispiel mit Hexan, Petroleum-ether, Diethylether verdünnen. Hierbei präzipitieren die Pluraflavine, die lipophilen Verunreinigungen bleiben gelöst und werden durch übliche Fest/flüssig-Phasentrennungen entfernt. Der Niederschlag, der die gesamten Pluraflavine enthält, wird in 1/30 des ursprünglichen Volumens Wasser/Methanol gelöst. Der Niederschlag geht dabei vollständig in Lösung und wird lyophilisiert. Das Lyophilisat, in der Folge als Rohprodukt bezeichnet, enthält 0,5 bis 50 % Pluraflavin und wird für die weitere Isolierung eingesetzt.

Die weitere Aufreinigung eines oder mehrerer der erfindungsgemäßen Pluraflavine erfolgt durch Chromatographie an geeigneten Materialien, vorzugsweise z.B. an Molekularsieben, an Normal-Phasenträger, wie z. B. Kieselgel, Aluminiumoxid, an lonenaustauschern oder an Adsorberharzen bzw. an Umkehr-Phasen (reversed phase, RP). Mit Hilfe dieser Chromatographie werden die Pluraflavine getrennt. Die Chromatographie der Pluraflavine erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, Propanol und Acetonitril, in einer Konzentration von 10 bis 80 % Lösemittel, vorzugsweise 40 bis 60 % Lösemittel oder auch alle gepufferten wässrigen Lösungen, die mit organischen Lösemitteln mischbar sind. Die zu verwendeten Puffer sind die gleichen wie oben angegeben.

Die Trennung der Pluraflavine aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxyd und ähnlichen erfolgen.

Die Chromatographie der Pluraflavine erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von 1 mM bis 0,5 M sowie Ameisensäure, Essigsäure, Trifluoressigsäure oder allen handelsüblichen, dem Fachmann bekannten Säuren, vorzugsweise in einer Konzentration von 0,01 bis 3 %, insbesondere 0,1 %.

Chromatographiert wird mit einem Gradienten, der mit 100 % wäßrigem Puffer beginnt und mit 100 % Lösemittel endet, vorzugsweise wird ein linearer Gradient von 10 bis 50 % 2-Propanol oder Acetonitril gefahren.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad), Fractogel TSK HW 40® (Fa. Merck, Deutschland oder Toso Haas, USA) oder an Sephadex® (Fa. Pharmacia, Uppsala, Schweden).

Die Reihenfolge der vorgenannten Chromatographien ist umkehrbar.

Ein weiterer, sehr wirksamer Reinigungsschritt für Pluraflavine ist die Kristallisation. Die Pluraflavine kristallisieren aus Lösungen in organischen Lösungsmitteln und aus Mischungen von Wasser mit organischen Lösungsmitteln. Die Kristallisation wird in an sich bekannter Weise, zum Beispiel durch Konzentrieren oder Abkühlen gesättigter Plurflavine-Lösungen, durchgeführt.

Die erfindungsgemäßen Pluraflavine sind im festen Zustand und in Lösungen im pH-Bereich zwischen 3 und 8, insbesondere 4 und 6, stabil und lassen sich damit in übliche galenische Zubereitungen einarbeiten.

Die Pluraflavine und abgeleitete chemische Derivate der Formel l können nach dem Fachmann bekannten Methoden in die entsprechenden physiologisch verträglichen Salzen übergeführt werden.

Unter physiologisch verträglichen Salzen von Verbindungen der Formel 1 versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung umfaßt weiterhin offensichtlich chemische Äquivalente der Verbindungen der Formel 1 die einen geringfügigen chemischen Unterschied aufweisen, also die gleiche Wirksamkeit haben oder sich unter milden Bedingungen in die erfindungsgemäße Verbindung umwandeln lassen. Zu den genannten Äquivalenten gehören z.B. Ester und Ether sowie Reduktionsprodukte der erfindungsgemäßen Verbindungen.

Ester, und Ether Derivate sowie Reduktionsprodukte können nach in der Literatur beschriebenen Verfahren, z.B., in "Advanced Organic Synthesis, 4^{th} Edition, J. March, John Wiley & sons., 1992, hergestellt werden.

Die vorliegende Erfindung umfaßt allen stereoisomeren Formen der Verbindungen der Formel 1. In den Verbindungen der Formel IA und IB enthaltene Asymmetriezentren können alle unabhängig voneinander die S-Konfiguration oder die R-Konfiguration aufweisen. Die Oxirane der Epoxid-enthaltendegruppe können in jede Positon liegen, bevorzugt sind die Oxirane mit der Beteilung der C-Atome 2' und 3'. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, R- und S-Konfigurationen, in Form von Racematen und in Form von Mischungen der beiden Enantiomeren in allen Verhältnissen Gegenstand der Erfindung. Bei Vorliegen einer cis/trans-Isomerie sind sowohl die *cis*-Form als auch die *trans*-Form und Gemische dieser Formen in allen Verhältnissen Gegenstand der Erfindung.

Aufgrund ihrer wertvollen pharmakologischen Eigenschaften eignen sich die erfindungsgemäßen Verbindungen zur Anwendung als Arzneimittel in der Human- und/oder Tiermedizin. Sie sind antibiotisch aktiv und besitzen neben der antibakteriellen Wirkung, antimykotische, d. h. pilzhemmende einschließlich der pflanzenpathogenen Pilze, antiprotozoische und antiparasitische Eigenschaften.

Die erfindungsgemäßen Verbindungen können gegen Krebserkrankungen verwendet werden, insbesondere als Chemotherapeutika. Aufgrund ihrer cytostatischen Eigenschaften, insbesondere ihrer stark antitumor Aktivität sowie einer antimikrobiellen Wirkung können sie insbesondere als Cytostatika gegen maligne Entartungen bei Tieren sowie beim Menschen eingesetzt werden.

Bei Tumorzellen, die Resistenzen gegen herkömmliche Mittel ausgebildet haben, besitzen nur neue Agentien eine therapeutisch ausreichende Wirkung. Die erfindungsgemäßen Pluraflavine und chemische Derivate davon der Formel I weisen somit potentiell eine hervorragende Wirkung auch gegen diese Problem-Zelltypen auf.

Die Erfindung betrifft auch pharmazeutische Zubereitungen, die eine oder mehrere der erfindungsgemäßen Pluraflavine und/oder chemische Derivate davon enthalten. Bevorzugt ist die Verwendung in Mischung mit geeigneten Hilfsstoffen oder Trägermaterial. Als Trägermaterial können beim Menschen alle pharmakologisch verträglichen Trägermaterialien und/oder Hilfsstoffe verwendet werden.

Die Erfindung betrifft auch ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk, Zusatz- oder Hilfsstoffen in eine geeignete Darreichungsform bringt.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Micheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Pluraflavine und/oder chemische Derivate davon enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,1 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1

### Herstellung einer Glycerinkultur von Actinomycetales species HAG 003959, DSM 12931.

100 ml Nährlösung (Malzextrakt 2,0%, Hefeextract 0,2 %, Glucose 1,0 % (NH₄)₂HPO₄ 0,05 %, pH 6,0) in einem sterilen 300 ml Erlenmeyerkolben werden mit dem Stamm *Actinomycetales* species HAG 003959, DSM 12931, beimpft und 7 Tage bei 28° C und 180 UpM auf einer rotierenden Schüttelmaschine inkubiert. 1,5 ml dieser Kultur werden anschließend mit 1,5 ml 99 %igem Glycerin verdünnt und bei -20° C gelagert.

### Beispiel 2

### Herstellung einer Kultur bzw. einer Vorkultur im Erlenmeyerkolben von Actinomycetales species HAG 003959, DSM 12931,

Ein steriler 300 ml Erlenmeyerkolben mit 100 ml der folgende Nährlösung: 15 g/l Glucose, 15 g/l Sojamehl, 5 g/l Cornsteep, 2 g/l CaCO₃ und 5 g/l NaCI wird mit einer auf einem Schrägröhrchen (gleiche Nährlösung, jedoch mit 2 % Agar) gewachsenen Kultur oder mit 1 ml einer Glycerinkultur (s. Beispiel 1) beimpft und auf einer Schüttelmaschine bei 180 UpM und 30 °C inkubiert. Die maximale Produktion einer oder mehrerer Verbindungen der erfindungsgemäßen Pluraflavine ist nach ca. 120 Stunden erreicht. Zum Animpfen von 10 und 200 1 Fermentern genügt eine 48 bis 96 Std. alte Submerskultur (Animpfmenge ca. 10 %) aus der gleichen Nährlösung.

### Beispiel 3

### Herstellung der Pluraflavine

Ein 9 I Fermenter wird unter folgenden Bedingungen betrieben:

| | |
|---|---|
| Nährmedium | 15 g/l Glucose; |
| | 15 g/l Sojamehl; |
| | 5 g/l Cornsteep, fest; |
| | 2 g/l CaCO_{3;} |
| | 5 g/l NaCI; |
| | pH 7.0 (vor der Sterilisation) |
| Prozessdauer | 92 Stunden |
| Inkubationstemperatur | 28° C |
| Rührergeschwindigkeit | 300 UpM |
| Belüftung | 5 I Min⁻¹ |

Durch wiederholte Zugabe von ethanolischer Polyollösung kann die Schaumbildung unterdrückt werden. Das Produktionsmaximum wird nach ca. 70 bis 96 Stunden erreicht.

### Beispiel 4

### Isolierung des Pluraflavin-Gemisches aus der Kulturlösung des Actinomycetales species HAG 003959, DSM 12931.

Nach Beendigung der Fermentation von *Actinomycetales* species HAG 003959, DSM 12931, wird die Kulturbrühe des Fermentern, gewonnen nach Beispiel 3, (90 Liter) unter Zusatz von ca. 2 % Filterhilfsmittel (z.B. Celite®) filtriert und die Zellmasse (0.6 Liter) mit 3 Liter Methanol extrahiert. Die Wirkstoff-haltige, methanolische Lösung wird durch Filtration vom Mycel befreit und im Vakuum konzentriert. Das Konzentrat wird gemeinsam mit dem Kulturfiltrat (7 Liter) auf eine vorbereitete, 0.4 Liter ®MCI GEL, CHP20P-Säule aufgetragen. Eluiert wird mit einem Gradienten von 0.1% Essigsäure in Wasser nach 0.1% Essigsäure in Propanol-2. Der Säulendurchfluß (1.2 Liter pro Stunde) wird fraktioniert aufgefangen (je 0.25 Liter) und die Pluraflavine enthaltenden Fraktionen (20 bis 23) zusammengefaßt. Konzentrieren im Vakuum und Gefriertrocknung ergeben 1.4 g eines braunen Pulvers.

### Beispiel 5

### Anreicherung der Pluraflavin-Komponenten durch Gel-Chromatographie.

1.4 g des nach Beispiel 4 gewonnenen Produktes werden auf eine 3,9 Liter fassende mit Fractogel® TSK HW-40 s gefüllte Säule (Breite x Höhe = 10 cm x 50 cm) aufgetragen. Das Laufmittel Wasser-Acetonitril (1:1) wird mit einer Flußrate von 50 ml pro Minute über die Säule gepumpt und der Säulenausfluß fraktioniert (65 ml) aufgefangen. Hauptsächlich in den Fraktionen 13 bis 16 befinden sich die Pluraflavine. Sie werden zusammengefaßt und im Vakuum vom Lösungsmittel befreit. Sie ergeben 130 mg Plurflavinegemisch.

### Beispiel 6

### Trennung der Pluraflavin-Komponenten auf Reverse Phase RP-18.

Es wird eine 122 ml fassende präparative HPLC-Säule (1.25 cm (ID) x 25 cm H) mit ®Nucleosil 100-7 C18 HD, gefüllt und die 130 mg des Pluraflavinegemisches, gewonnen nach Beispiel 5, aufgetragen. Eluiert wird mit 10 % Acetonitril in 0,1 M wässriger Ammoniumacetat-Lösung. Der Säulendurchfluß beträgt 50 ml / Minute, es werden Fraktionen von je 50 ml Inhalt gesammelt. In den Fraktion 12-17 befindet sich das Pluraflavin C, in Fraktionen 25 bis 27 das Pluraflavin B und in den Fraktionen 35 bis 37 das Pluraflavin A. Nach dem Konzentrieren im Vakuum und Gefriertrocknung werden folgende Mengen ausgewogen:
Pluraflavin A: 22 mg, ESI+ MS: 823 Da ( M+H )⁺,
Pluraflavin B: 18 mg, ESI+ MS: 841 Da ( M+H )⁺,
Pluraflavin C:11 mg, ESI+ MS: 974 Da ( M+H )⁺,

### Beispiel 7

### Endreinigung des Pluraflavin A und Überführung in die Trifluoracetatsalz-Form.

Die Fraktionen 35 bis 37, gewonnen nach Beispiel 6, wird nach Gefriertrocknung (22 mg), gelöst in 10% Acetonitril in Wasser, mit Trifluoressigsäure auf pH 2,8 eingestellt, auf eine 250/10 LiChrospher RP-18e (5µm)® - Säule aufgetragen. Die Elution erfolgt mit 0,05% wäßriger Trifluoressigsäure in 11 bis 22% Acetonitril. Nach Konzentrieren im Vakuum und Gefriertocknung werden 18 mg Pluraflavin A - Trifluoracetatsalz gewonnen.

### Beispiel 8

### Charakterisierung des Pluraflavin A.

Aussehen: in polaren organischen Lösungsmitteln lösliche, jedoch in Wasser nur wenig lösliche, tiefgelbe Substanz. Die Säure-Additionssalze sind in Wasser löslich. Die Verbindung ist stabil im neutralem und mild-saurem Milieu, jedoch unbeständig im alkalischen und stark-saurem Bereich.
UV-Maxima: 214, 243, 270 (Sh), 290 (Sh), 426 nm in Wasser-Acetonitril (8:2), pH 2 sowie 214, 243, 270 (Sh), 290 (Sh) und 426 nm in Wasser-Acetonitril (6:4), pH 7.
IR-Banden: 3424, 1680, 1600, 1464, 1427, 1300, 1203, 1131, 1066, 1009, 801, 721 cm⁻¹.
Durch hochauflösende Massenspektrometrie wurde für (M + H)⁺ folgendes Molekulargewicht gefunden: 823,370631 Da, entsprechen der Summenformel für das Pluraflavin A von C₄₃H₅₄N₂O₁₄. Durch Elektronenspray ― lonisation (ESI, positiv) werden mittels MS/MS-Fragmentierung folgende lonen erhalten: 823, 693, 680, 550, 480, 390, 320, 144 und 131 Da.
NMR-Signale: siehe Tabelle 1.

**Tabelle 1:**

| ¹H- und ¹³C- chemische Verschiebungen von Pluraflavin A in Methanol-d₄ und DMSO-d₆ bei 300K | | | | |
|---|---|---|---|---|
| | **Methanol-d**_{**4**} | | **DMSO-d**_{**6**} | |
| **Position** | ^{**13**}**C** **□ (ppm)** | ^{**1**}**H** **□ (ppm)** | ^{**13**}**C** **□ (ppm)** | ^{**1**}**H** **□ (ppm)** |
| 2 | 168.39 | - | 165.62 | - |
| 3 | 111.95 | 6.37 s | 110.39 | 6.27 s |
| 4 | 180.25 | - | 177.56 | - |
| 4a | 126.08 | - | 124.33 | - |
| 5 | 150.44 | - | 148.22 | - |
| 6 | 121.34 | 8.61 s | 119.05 | 8.43s |
| 6a | 138.34 | - | 136.28 | - |
| 7 | 182.68 | - | 181.12 | - |
| 7a | 133.23 | - | 131.18 | - |
| 8 | 120.05 | 7.86 d | 118.29 | 7.78 d |
| 9 | 135.63 | 7.90 d | 134.41 | 7.87 d |
| 10 | 137.35 | - | 135.79 | - |
| 11 | 161.06 | - | 159.39 | - |
| 11-OH | - | - | - | 13.38 s, br |
| 11a | 118.14 | - | 116.43 | - |
| 12 | 189.12 | - | 187.39 | - |
| 12a | 122.00 | - | 120.35 | - |
| 12b | 157.66 | - | 155.50 | - |
| 13 | 70.83 | 5.38 d, 5.63 d | 68.48 | 5.30 d, 5.51 d |
| 14 | 61.17 | - | 59.35 | - |
| 15 | 63.69 | 3.41 q | 61.61 | 3.48 q |
| 16 | 20.24 | 1.92 s | 19.32 | 1.86 s |
| 17 | 13.69 | 1.27 d | 12.93 | 1.22 d |
| 1' | 98.90 | 5.04 dd | 96.57 | 5.06 d, br |
| 2' | 37.24 | 2.05, 2.12 | 35.50 | 1.87, 1.95 |
| 3' | 58.32 | - | 56.33 | - |
| 3'Me | 24.57 | 1.50 s | | 1.35 s |
| 3'NH₂ | - | - | - | 8.16 s, br |
| 4' | 71.15 | 3.27 s, br | 68.65 | 3.17 d |
| 4'-OH | - | - | - | 5.47 d |
| 5' | 70.44 | 4.00 q | 68.19 | 4.01 q |
| 6' | 17.21 | 1.34 d | 16.75 | 1.18 d |
| 1" | 70.23 | 5.51 t | 68.21 | 5.44 br |
| 2" | 28.00 br | 2.48 m, 2.91 m,br | | 2.35 br, 2.78 br |
| 3" | 65.19 | 3.45 m, br | 62.19 | 3.43 br |
| 3"NMe | 43.24 br, 41.65 br | 3.05 br, 3.05 br | 42.14, 39.25 | 2.89, 2.97 |
| 3"NH⁺ | - | - | - | 8.78 br |
| 4" | 75.13 | 4.32 s, br | 71.88 | 4.20 s, br |
| 5" | 72.55 | 3.97 q, br | 70.22 | 3.75 q, br |
| 6" | 18.09 | 1.40 d | 17.54 | 1.28d |
| 1"' | 101.39 | 5.24 m, br | 98.89 | 5.11 br |
| 2"' | 33.40 | 2.05, 2.10 | 32.07 | 1.75, 1.92 |
| 3"' | 66.61 | 4.12 m | 64.17 | 3.96 m, br |
| 3"'-OH | - | - | - | 4.70 s, br |
| 4"' | 71.98 | 3.67 | 70.13 | 3.45 br |
| 4"'-OH | - | - | - | 4.44 s, br |
| 5"' | 69.52 | 4.16 q, br | 67.05 | 4.10 q, br |
| 6"' | 17.51 | 1.28 d | 16.94 | 1.10 d |

Für die Aldohexose III stehen die beobachteten NOEs in Einklang mit einer relativen Stereochemie SRSS bzw. RSRR der chiralen Zentren 1', 3', 4', 5' (Abb. 1). Dies entspricht dem C-3-Epimer des Vancosamins. 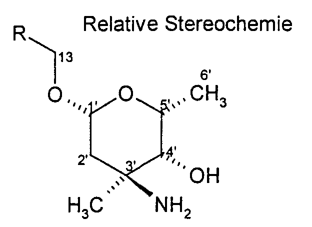 Abb. 1 Relative Stereochemie der Hexose III.

Die für die Hexose II beobachteten NOE-Effekte favorisieren die relative Stereochemie RSSS bzw. SRRR für die chiralen Zentren 1", 3", 4", 5" (Abb. 2). 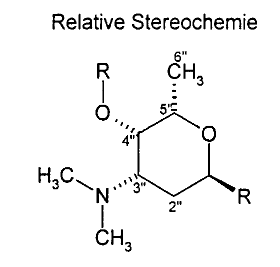 Abb. 2 Relative Stereochemie der Hexose II.

Die gleiche relative Stereochemie (RSSS, SRRR) für die asymmetrischen Kohlenstoffe 1''', 3"', 4"', 5"' steht mit den detektierten NOEs der Hexose I in Einklang (Abb. 3). 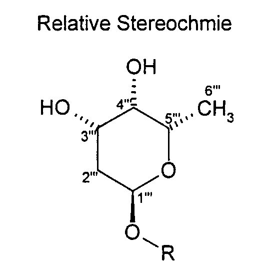 Abb. 3 Relative Stereochemie der Hexose I.

### Beispiel 9

### Charakterisierung des Pluraflavin B.

Aussehen: in polaren organischen Lösungsmitteln lösliche, jedoch in Wasser nur wenig lösliche, tiefgelbe Substanz. Die Säure-Additionssalze sind in Wasser löslich. Die Verbindung ist stabil im neutralem und mild-saurem Milieu, jedoch unbeständig im alkalischen Bereich.
UV-Maxima: 214, 243, 270 (Sh), 290 (Sh), 426 nm in Wasser-Acetonitril (8:2), pH 2 sowie 214, 243, 270 (Sh), 290 (Sh) und 426 nm in Wasser-Acetonitril (6:4), pH 7. Pluraflavin B hat die Summenformel C₄₃H₅₆N₂O₁₅, das Molekulargewicht ist 840,9 Da.
NMR-Signale: siehe Tabelle 2.

**Tabelle 2:**

| ¹H- und ¹³C- chemische Verschiebungen von Pluraflavin B in Methanol-d₄ und Vergleich mit Pluraflavin A bei 300K | | | | |
|---|---|---|---|---|
| | **Pluraflavin B (Methanol-d**_{**4**}**)** | | **Pluraflavin A (Methanol-d**_{**4**}**)** | |
| **Position** | ^{**13**}**C** **□ (ppm)** | ^{**1**}**H** **□ (ppm)** | ^{**13**}**C** **□ (ppm)** | ^{**1**}**H** **□ (ppm)** |
| 2 | 176.38 | - | 168.39 | - |
| 3 | 111.10 | 6.73 s | 111.95 | 6.37 s |
| 4 | 181.26 | - | 180.25 | - |
| 4a | 125.96 | - | 126.08 | - |
| 5 | 150.43 | - | 150.44 | - |
| 6 | 121.03 | 8.52 s | 121.34 | 8.61 s |
| 6a | 138.12 | - | 138.34 | - |
| 7 | 182.66 | - | 182.68 | - |
| 7a | 133.16 | - | 133.23 | - |
| 8 | 120.04 | 7.81 d | 120.05 | 7.86 d |
| 9 | 135.52 | 7.83 d | 135.63 | 7.90 d |
| 10 | 137.44 | - | 137.35 | - |
| 11 | 161.02 | - | 161.06 | - |
| 11-OH | - | - | - | - |
| 11a | 118.01 | - | 118.14 | - |
| 12 | 189.24 | - | 189.12 | - |
| 12a | 121.89 | - | 122.00 | - |
| 12b | 157.41 | - | 157.66 | - |
| 13 | 70.83 | 5.36 d, 5.60 d | 70.83 | 5.38 d, 5.63 d |
| 14 | 77.67 | - | 61.17 | - |
| 15 | 72.61 | 4.40 q | 63.69 | 3.41 q |
| 16 | 23.90 | 1.67 s | 20.24 | 1.92 s |
| 17 | 17.00 | 1.35 d | 13.69 | 1.27 d |
| 1' | 98.87 | 5.05 dd | 98.90 | 5.04 dd |
| 2' | 37.20 | 2.09 m | 37.24 | 2.05, 2.12 |
| 3' | 58.39 | - | 58.32 | - |
| 3'Me | 24.45 | 1.51 s | 24.57 | 1.50 s |
| 3'NH₂ | - | - | - | - |
| 4' | 71.08 | 3.29 s, br | 71.15 | 3.27 s, br |
| 4'-OH | - | - | - | - |
| 5' | 70.42 | 4.01 q | 70.44 | 4.00 q |
| 6' | 17.19 | 1.33 d | 17.21 | 1.34d |
| 1" | 70.13 | 5.48 t | 70.23 | 5.51 t |
| 2" | 27.74 | 2.50 m, 2.88 m | 28.00 br | 2.48 m, 2.91 m,br |
| 3" | 65.14 | 3.46 m | 65.19 | 3.45 m, br |
| 3"NMe | 43.30, 41.40 | 3.01 s, 3.12 s | 43.24 br, 41.65 br | 3.05 br, 3.05 br |
| 3"NH⁺ | - | - | - | - |
| 4" | 75.02 | 4.32 s, br | 75.13 | 4.32 s, br |
| 5" | 72.61 | 3.97 q, br | 72.55 | 3.97 q, br |
| 6" | 18.15 | 1.39 d | 18.09 | 1.40 d |
| 1''' | 101.38 | 5.24 m, br | 101.39 | 5.24 m, br |
| 2"' | 33.42 | 2.07 m | 33.40 | 2.05, 2.10 |
| 3"' | 66.59 | 4.13 m | 66.61 | 4.12 m |
| 3"'-OH | - | - | - | - |
| 4"' | 71.97 | 3.67 br | 71.98 | 3.67 |
| 4"'-OH | - | - | - | - |
| 5"' | 69.52 | 4.16 q, br | 69.52 | 4.16 q, br |
| 6"' | 17.49 | 1.27 d | 17.51 | 1.28 d |

### Beispiel 10

### Untersuchung der cytostatischen Wirksamkeit.

(a) Zur Bestimmung der cytostatischen Aktivität wurde mit Ratten-Hepatomazellen gearbeitet, die von der Stammsammlung American Type Culture Collection unter der Nummer ATCC CRL-1548, Jo Nr. 223 und 228 beschafft worden sind. Die Stammhaltung geschah im "MEM (EAGLE) with Glutamax"-Medium [Fa. GIBCO BRL N° 4109-028] mit 10% fötalem Kälberserum [Fa. GIBCO BRL N° 10270-106] und 10 µl/ml Penicillin-Streptomycin [Fa. GIBCO BRL N° 15140-114]. Gearbeitet wurde in 96-er Mikrotiterplatten [Fa. Greiner, N° 15140-114]. Je Vertiefung (Well) wurden 140 µl Stammhaltungs-Nährlösung vorgelegt und mit je 215.000 Zellen eingesät. Hiernach wurde 20-24 Stunden bei 37° C und 5% CO₂ inkubiert. Eine vorher vorbereitete Verdünnungsreihe der Pluraflavine mit Konzentrationen von 100, 50, 25, 12.5, 6.25, 3.125, 1.5625, 0.7813, 0.3906, 0.195, 0.094, 0.047 und 0 µM wurde dann in der entsprechenden Reihenfolge pipettiert. Nach einer weiteren Inkubationszeit von 22 Stunden bei 37° C in einer Atmosphäre von 5% CO₂ ist dann das flüssige Medium abgesaugt worden. Die Anfärbung der zurückbleibenden Zellen wurden mit 100 µl/Well RPMI 1640 [Fa. GIBCO BRL N° 32404-014] und 20 µl/Well Cell Titer 96 Aqueous [Fa. PROMEGA N° G5430] durchgeführt. Die Messung der Lichtabsorption geschah bei 590 nm direkt nach Zugabe und nach 2 Stunden Inkubation, wie oben. Aus der Veränderung der Lichtabsorption wurde die cytostatische Wirkung berechnet.
IC₅₀ für Pluraflavin A = < 50 nM;
IC₅₀ für Pluraflavin B = < 50 nM.

## Patentansprüche

1. Verbindung der allgemeinen Formel I 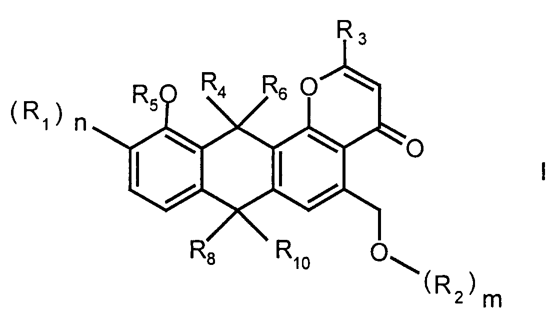 worin bedeuten
R₁ und R₂ unabhängig von einander jeweils einen Zucker,
R₃ eine Epoxid-enthaltende Gruppe, oder
C₁-C₆-Alkyl oder C₂-C₆-Alkenyl, gegebenenfalls mit einem oder mehreren OH substituiert,
R₅ H, C₁-C₆-Alkyl, C₂-C₆-Alkenyl oder C₂-C₆-Alkinyl,
R₄, R₆, R₈ und R₁₀, unabhängig von einander, H, C₁-C₆-Alkyl, C₂-C₆-Alkenyl, C₂-C₆-Alkinyl, -X₂H, oder -X₂R₁₂, oder R₄ und R₆ zusammen und/oder R₈ und R₁₀ zusammen =X₂,
X₂ O, NH, N-C₁-C₆-Alkyl, N-C₂-C₆-Alkenyl, N-C₂-C₆-Alkinyl oder S,
R₁₂ C₁-C₆-Alkyl, C₂-C₆-Alkenyl, C₂-C₆-Alkinyl, Aryl oder Acyl, und
m und n 1 oder 2, in allen ihren stereochemischen Formen und Gemische dieser Formen in jedem Verhältnis,
sowie deren physiologisch verträglichen Salze.

2. Verbindung der Formel I gemäß Anspruch 1, worin R₂ einen Aminozucker bedeutet.

3. Verbindung der Formel 1 gemäß Anspruch 1 oder 2, worin R₂ die Formel 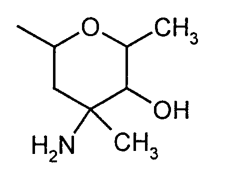 hat.

4. Verbindung der Formel I gemäß einem oder mehreren der Ansprüche 1 - 3, worin n 2 bedeutet.

5. Verbindung der Formel 1 gemäß einem oder mehreren der Ansprüche 1 - 4, worin R₁ einen Aminozucker bedeutet.

6. Verbindung der Formel I gemäß einem oder mehreren der Ansprüche 1-5, worin R₁ die Formel 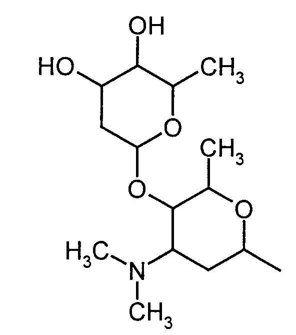 hat.

7. Verbindung der Formel 1 gemäß einem oder mehreren der Ansprüche 1 - 6, worin R₃ die Formel 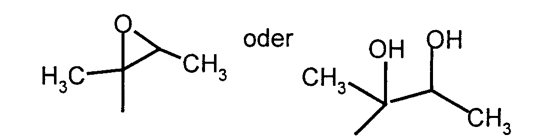 hat.

8. Verbindung der Formel IA 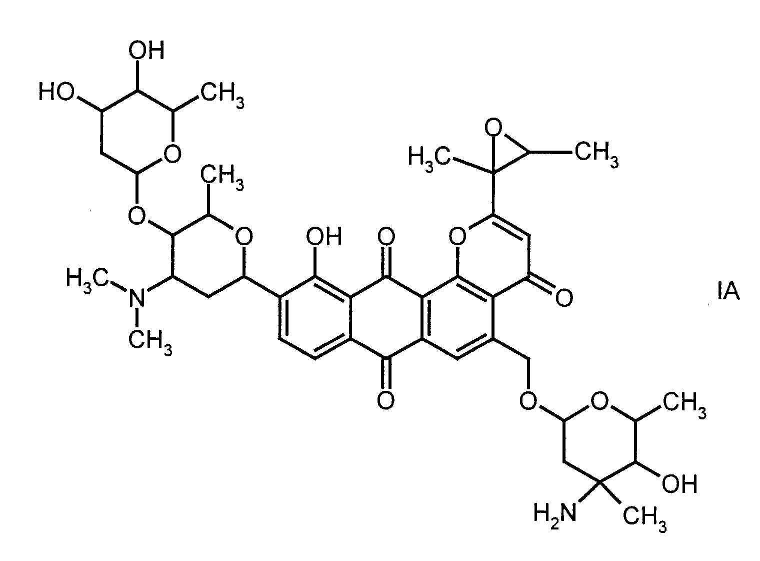 in allen ihren stereochemischen Formen und Gemische dieser Formen in jedem Verhältnis, sowie deren physiologisch verträglichen Salzen

9. Verbindung der Formel IB 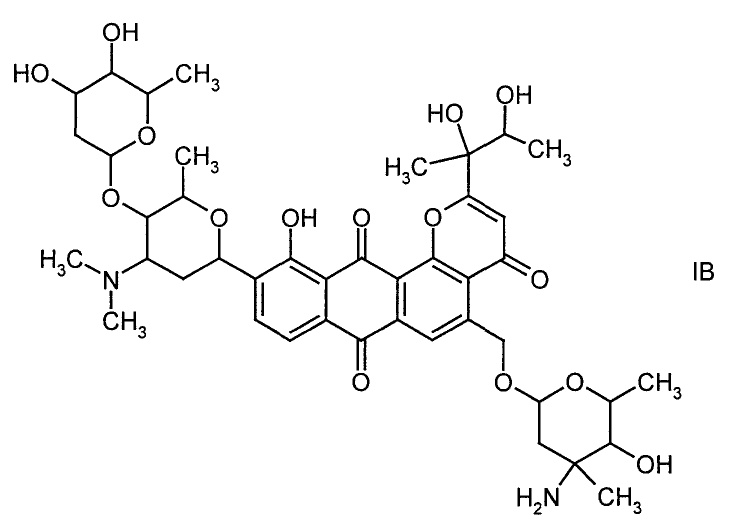 in allen ihren stereochemischen Formen und Gemische dieser Formen in jedem Verhältnis, sowie deren physiologisch verträglichen Salzen.

10. Verbindung der Formel I gemäß einem oder mehreren der Ansprüche 1 -9, erhältlich durch Fermentierung von *Actinomycetales species* HAG 003959, DSM 12931, oder einer seiner Varianten oder Mutanten unter geeigneten Bedingungen in einem Kulturmedium, bis sich eine Verbindung der Formel IA und/oder IB in dem Kulturmedium anhäuft und durch anschließende Isolierung der Verbindung und gegebenenfalls Umwandlung in chemische Derivate, sowie deren physiologisch verträglichen Salze.

11. Verfahren zur Herstellung einer Verbindung der Formel 1 oder ein physiologisch verträgliches Salz davon gemäß einem oder mehreren der Ansprüche 1 - 10, dadurch gekennzeichnet, daß der Mikroorganismus *Actinomycetales* species HAG 003959, DSM 12931, oder einer seiner Varianten oder Mutanten unter geeigneten Bedingungen in einem Kulturmedium fermentiert wird, bis sich eine Verbindung der Formel IA und/oder IB in dem Kulturmedium anhäuft und anschließend aus dem Kulturmedium isoliert und gegebenenfalls in chemische Derivate und physiologisch verträglichen Salze umgewandelt werden.

12. Verfahren gemäß Anspruch 11, dadurch gekennzeichnet, daß die Fermentation unter aeroben Bedingungen bei einer Temperatur zwischen 18 und 35°C und bei einem pH zwischen 6 und 8 durchgeführt wird.

13. Verfahren gemäß Anspruch 11 oder 12 , dadurch gekennzeichnet, daß die Verbindung der Formel IA oder IB mit einem Reduktionsmittel in ein chemisches Derivat umgewandelt wird.

14. Verbindung der Formel 1 oder ein physiologisch verträgliches Salz davon gemäß einem oder mehreren der Ansprüche 1 - 10 zur Verwendung als Arzneimittel.

15. Verbindung der Formel 1 oder ein physiologisch verträgliches Salz davon gemäß einem oder mehreren der Ansprüche 1 - 10 zur Verwendung alsArzneimittel zur Hemmung von Transkriptasen.

16. Verbindung der Formel I oder ein physiologisch verträgliches Salz davongemäß einem oder mehreren der Ansprüche 1 - 10 zur Verwendung als Cytostatikum.

17. Pharmazeutische Zubereitung mit einem Gehalt an mindestens einer Verbindung der Formel 1 oder ein physiologisch verträgliches Salz davon gemäß einem oder mehreren der Ansprüche 1-10.

18. Verfahren zur Herstellung von pharmazeutischen Zubereitungen gemäß Anspruch 17, dadurch gekennzeichnet, daß man mindestens eine Verbindung der Formel 1 oder ein physiologisch verträgliches Salz davon gemäß einem oder mehreren der Ansprüche 1-10 mit geeigneten Hilfs- und/oder Trägerstoffen in eine geeignete Darreichungsform bringt.

19. *Actinomycetales species* HAG 003959 (DSM 12931).
